# EUROPEAN PATENT SPECIFICATION

(11) **EP 2 194 116 B1**
(45) Date of publication and mention of the grant of the patent: **06.08.2014**
(21) Application number: 08828531.7
(22) Date of filing: 22.08.2008
(51) Int. Cl.: C12N 15/11, C12N 15/87

(54) **Transfection device utilizing sericin hydrolysate**
Transfektionsvorrichtung mit Sericinhydrolysat
Dispositif de transfection avec hydrolysat de séricine

(30) Priority: 24.08.2007 JP 2007218733
(43) Date of publication of application: 09.06.2010
(73) Proprietor: CytoPathfinder, Inc., Tokyo 103-0022 (JP)
(72) Inventor: FUJITA, Yoshiji, Tokyo 103-0022 (JP); YOSHIKAWA, Tomohiro, Tokyo 103-0022 (JP)
(74) Representative: Hofmann, Andreas
(86) International application number: PCT/JP2008/065027
(87) International publication number: WO 2009/028421

(56) References cited:
- EP-A1- 1 302 544
- JP-A- 11 243 948
- JP-A- 2003 509 060
- JP-A- 2006 511 229
- US-B2- 6 905 878
- MINOURA N ET AL: "ATTACHMENT AND GROWTH OF CULTURED FIBROBLAST CELLS ON SILK PROTEIN MATRICES", JOURNAL OF BIOMEDICAL MATERIALS RESEARCH, WILEY, NEW YORK, NY, US, vol. 29, no. 10, 1 January 1995 (1995-01-01), pages 1215-1221, XP001106174, ISSN: 0021-9304, DOI: 10.1002/JBM.820291008
- DATABASE WPI Thomson Scientific, London, GB; AN 1999-564429 XP002689822, "Cell culture bed base material for animal proliferation", & JP 11 243949 A (INOUE K. ET AL.) 14 September 1999 (1999-09-14)
- ZIAUDDIN J AND SABATINI DM: 'Microarrays of cells expressing defined cDNAs' NATURE vol. 411, 2001, pages 107 - 110, XP002963625
- WU RZ ET AL: 'Cell-biological applications of transfected-cell microarrays' TRENDS CELL BIOL. vol. 12, 2002, pages 485 - 488, XP001184158
- UCHIMURA E ET AL: 'On-chip transfection of PC12 cells based on the rational understanding of the role of eCM molecules: efficient, non-viral transfection of PC12 cells using collagen IV' NEUROSCI. LETT. vol. 378, 2005, pages 40 - 43, XP004778690
- YOSHIKAWA T ET AL: 'Transfection microarray of human mesenchymal stem cells and on-chip siRNA gene knockdown' J. CONTROL RELEASE vol. 96, 2004, pages 227 - 232, XP004502172
- OCHIYA T ET AL: 'New delivery system for plasmis DNA in vivo using atelocollagen as a carrier material' NAT. MED. vol. 5, 1999, pages 707 - 710, XP008131971
- HONMA K ET AL: 'Atelocollagen-based gene transfer in cells allows high-throughput screening of gene functions' BIOCHEM. BIOPHYS. RES. COMMUN. vol. 289, 2001, pages 1075 - 1081, XP002957375

### SEQUENCE LISTING

<110> cytopathfinder. Inc.
<120> transfection device
<130> B07-0296
<160> 5
<170> PatentIn version 3.1
<210> 1
   <211> 19
   <212> RNA
   <213> Homo sapiens
<400> 1
   gaaaugaaca ggagucaau 19
<210> 2
   <211> 19
   <212> RNA
   <213> Homo sapiens
<400> 2
   gcaagaggau cggguguau 19
<210> 3
   <211> 19
   <212> RNA
   <213> Homo sapiens
<400> 3
   ucugcuggug cuacauaca 19
<210> 4
   <211> 19
   <212> RNA
   <213> Homo sapiens
<400> 4
   ggaacaagga cagggagau 19
<210> 5
   <211> 19
   <212> RNA
   <213> Homo sapiens
<400> 5
   caccaaugcu uccaacaau 19

## Description

### TECHNICAL FIELD

The present invention relates to a transfection device for transferring an exogenous gene sample (for example, single-stranded or double-stranded deoxyribonucleic acid (DNA), ribonucleic acid (RNA), aptamer and a chemically modified derivative thereof) into an animal cell.

### BACKGROUND ART

A device for transfection (gene transfer) having a gene sample immobilized on a solid-phase support can be used for various applications, such as gene therapy and gene analysis. As transfection device techniques that have been reported, there can be mentioned a technique in which a gene sample is immobilized with Extra Cellular Matrix (ECM) molecule, such as gelatin and fibronectin (see Patent Documents 1,2 or Non-Patent Document 1), and a technique in which a gene sample, together with calcium phosphate salt, is deposited on a solid-phase surface (see Non-Patent Documents 2 to 6).
Patent Document 1: U. S. Patent No. 6,544,790
Patent Document 2: U. S. Patent No. 6,905,878
Non-Patent Document 1: J.Ziauddin and D.M.Sabatini. Microarrays of cells expressed defined cDNAs. Nature 411, 107-110 (2001).
Non-Patent Document 2: R.Z.Wu et al. Cell-biological applications of transfected-cell microarrays. Trends in Cell Biology 12, 485-488 (2002).
Non-Patent Document 3: T.Ochiya et al. New delivery system for plasmid DNA in vivo using atelocollagen as a carrier material: the Minipellet. Nature Med. 5, 707-710 (1999)
Non-Patent Document 4: K.Honma et al. Atelocollagen-based gene transfer in cells allows high-throughput screening of gene functions. Biochem.Biophys.Res.Commu. 289, 1075-1081 (2001)
Non-Patent Document 5: T.Yoshikawa et al. Transfection microarray of human mesenchymal stem cells and on-chip siRNA gene knockdown. J.Controlled Release 96, 227-232 (2004).
Non-Patent Document 6: E.Uchimura et al. On-chip transfection of PC 12 cells based on the rational understanding of the role of ECM molecules: efficient, non-viral transfection of PC12 cells using collagen IV. Neuroscience Lett. 378, 40-43 (2005).

### DISCLOSURE OF THE INVENTION

However, in the conventional transfection device techniques described in the above-mentioned documents, ECM molecule and derivatives thereof are required, in addition to the gene sample and the gene delivery material. The ECM molecule, such as fibronectin and collagen, includes molecules that directly affect cell functions, by binding with an integrin receptor on a surface of a cell and inputting an external stimulus to the cell. Therefore, in the case of the transfection device using ECM molecule, there is a concern that cell functions (such as differentiation induction, increase or suppression of proliferation) other than gene transfer may be triggered as a side effect. In the transfection device to be used for examining effects of gene sample transfer on cell functions, it is desired to use materials causing no side effects.

The present invention was made under such a circumstance, and the object is to provide a novel transfection device for performing transfection with high gene material transfer efficiency and high reproducibility, with fewer side effects.

For attaining the object above, in a first aspect of the present invention, the transfection device has a mixture immobilized on a solid-phase support, the mixture including: a gene sample; a gene delivery material; and a sericin hydrolysate having a molecular weight of approximately 5 000 to 40 000.

### <Action and effect>

The transfection device of the present invention exhibits equivalent or higher gene transfer efficiency and reproducibility, as compared with the conventional transfection derive in which the gene sample, the gene delivery material and the ECM molecule or mammal-derived component are immobilized on the solid-phase support. In addition, in the conventional transfection device, there is a concern that the solid-phase support containing ECM molecule or mammal-derived component may cause cell differentiation or proliferation (hereinafter, referred to as "side effect"). On the other hand, in the present invention, neither ECM molecule nor mammal-derived component is used, and thus a solid-phase surface which causes few side effects can be provided. With this feature, utility and versatility of the transfection device are expected to be enhanced. Moreover, sericin is a water-soluble component obtained during refining of silk, and thus no change in activities thereof occurs even after autoclave/heat sterilization. On the other hand, the ECM molecule or mammal-derived component loses bioactivities after autoclave/heat sterilization, and thus differs widely from the sericin compound (sericin, hydrolysate thereof or a chemically modified product of sericin or a hydrolysate thereof). Furthermore, due to its antioxidant effect and cell-protective effect, the sericin compound is expected to alleviate cytotoxicity which may otherwise be caused during transfection.

Another advantage lies in that, while the ECM molecule requires complex processes for its purification and thus is very costly, the present invention uses the sericin compound which is relatively easily separated and purified from a water-soluble component during refining process, and thus the transfection device exhibiting equivalent or higher performance can be provided at a low price.

It should be noted that through intensive and extensive studies the present inventors elucidated the following knowledge for the first time: by immobilizing, on the solid-phase support, a sericin hydrolysate having a molecular weight of approximately 5 000 to 40 000, in addition to the gene sample and the gene delivery material, it becomes possible to measure effects of gene sample transfer on cell functions, without being interfered by, for example, differentiation induction, increase or suppression of proliferation, which may otherwise be caused by the presence of ECM molecule, and thus the utility and versatility of the transfection device are enhanced.

In a second aspect of the present invention, the gene sample is at least one member selected from the group consisting of single-stranded or double-stranded deoxyribonucleic acid, ribonucleic acid, aptamer, and a chemically modified derivative thereof.

### <Action and effect>

When the single-stranded or double-stranded deoxyribonucleic acid or ribonucleic acid is used as gene sample, overexpression of gene, antisense effect, and RNA interference or the like is expected. Accordingly, it becomes possible to analyze a gene function. It is expected that these chemical modifications of nucleic acid lead to stabilization and nonspecific reaction of nucleic acids.

When the aptamer is used as gene sample, a functional inhibition can be obtained utilizing its strong binding with a target protein. Especially in a case of the chemically-modified aptamer, it provides, for example, stabilization and suppression of a nonspecific reaction, as well as specific labeling of the target protein.

In a thrid aspect of the present invention, the gene delivery material includes at least one member selected from the group consisting of a cationic polymer, a cationic lipid, and a mineral.

### <Action and effect>

A gene delivery material is a material for inducing endocytosis, pinocytosis, phagocytosis, and fusion with cell membranes, upon transferring the gene sample into a cell. When the gene delivery material contains at least one member selected from the group consisting of a cationic polymer, a cationic lipid, and a mineral, the gene sample can be efficiently transferred into a cell.

In a fourth aspect of the present invention, the mixture further includes one of the followings: at least one compound selected from the group consisting of glucose, sucrose, glycogen, glutamine, arginine, histidine, lysine, threonine, tryptophan, valine, alanine, glycine, proline, and serine; a medium component for culturing cell; and pure water.

### <Action and effect>

As mentioned above, when the mixture further including one of the followings: at least one compound selected from the group consisting of glucose, sucrose, glycogen, glutamine, arginine, histidine, lysine, threonine, tryptophan, valine, alanine, glycine, proline, and serine; a medium component for culturing cell; and pure water, is immobilized on the solid-phase support, gene transfer efficiency can be enhanced.

As compared with the conventional transfection device in which the gene sample, the gene delivery material, and the ECM molecule are immobilized on the solid-phase support, the transfection device of the present invention can perform the gene transfer approximately 2 to 10 times as efficiently (though it varies from cell to cell), and as a result, high reproducibility can be secured. Accordingly, the utility of the transfection device is expected to be enhanced.

In a fifth aspect of the present invention, a material of the solid-phase support is selected from the group consisting of glass, a synthetic polymer, a metal, and a natural polymer.

### <Action and effect>

In a case where the solid-phase support is made from a hard plate (e.g., glass, plastic, and metal), it provides an appropriate environment as a support for cell array. Especially in a case of a support having a high light permeability property, a microscopic observation is easily performed, while in a case of a conductive support made of metal, it becomes possible to measure a potential change.

In a case where the support is made from a soft plate (e.g. polymer film), it can provide a transfection device in a shape of a sheet, or is applicable to a patch support or the like.

In a case where the support is in a form of particle or polymer matrix, it is applicable to an in vivo delivery, controlled-release support or the like.

### BRIEF DESCRIPTION OF THE DRAWINGS

Fig. 1 is a diagram illustrating one example of a method for fabricating a transfection device (glass array) of the present invention using a glass slide as solid-phase support.
Fig. 2 is a diagram schematically illustrating one example of a method for fabricating a transfection device of the present invention using a multiwell plate as solid-phase support.
Fig. 3 shows solution compositions of 56 samples used in Example 1.
Fig. 4 shows a fluorescence image of the transfection device of the present invention.
Fig. 5 illustrates a summary of a procedure for performing transfection using a plasmid DNA transfection device of the present invention.
Fig. 6 shows a fluorescence image (a) and a phase-contrast image (b) with respect to the plasmid DNA transfection device of the present invention.
Fig. 7 illustrates a summary of a procedure for performing transfection using an siRNA transfection device of the present invention.
Fig. 8 is a graph showing a result of transfection using the siRNA transfection device of the present invention.
Fig. 9 illustrates a summary of a procedure for performing transfection using the siRNA transfection device of the present invention.
Fig. 10 is a graph showing a result of transfection using the siRNA transfection device of the present invention (measurement result of an inhibition effect of siRNA transfer on TRAIL-induced apoptosis).
Fig. 11 shows solution compositions of 6 samples used in Example 5.
Fig. 12 is a graph showing a result of transfection using the siRNA transfection device of the present invention (retention of an activity of a sericin hydrolysate solution after heat sterilization).
Fig. 13 shows a solution composition of a mixed solution used in Example 6.
Fig. 14 is a graph showing a result of transfection using the siRNA transfection device of the present invention (alleviating effect of sericin on cytotoxicity).

### BEST MODE FOR CARRYING OUT THE INVENTION

Before describing the embodiments of the present invention, terms used in the claims and the specification will be explained below.

### (Gene sample)

Examples of the gene sample to be used in the present invention include: single-stranded or double-stranded deoxyribonucleic acid (e.g., plasmid DNA), ribonucleic acid (e.g., siRNA), aptamer, and a chemically modified derivative of these.

### (Gene delivery material)

Examples of the gene delivery material to be used in the present invention include: a cationic polymer, a cationic lipid and a mineral. These can be used alone or optionally in combination.

The "hydrolysate of sericin" to be used in the present invention means a peptide or polypeptide having a gene transfer ability, such as: a mixture of peptides or polypeptides having various molecular weights obtained by hydrolysis of natural sericin using an appropriate acid, alkali, enzyme or the like; a peptide or polypeptide having a specific amino acid sequence obtained when natural sericin is hydrolyzed; and a peptide or polypeptide having such a specific amino acid sequence artificially obtained by chemical synthesis or genetic engineering.

It should be noted that, in the present invention, a sericin hydrolysate has a molecular weight of approximately 5,000 to 40,000.

Examples of the chemically modified product of the hydrolysate thereof include: a sericin hydrolysate having an appropriate substituent (alkyl group or the like) introduced thereinto; and a sericin hydrolysate thereof in which an amino acid sequence has deletion, substitution, insertion or addition of one or more of amino acid residue. The chemically modified product is not limited to these, as long as it has a gene transfer ability.

### (Solid-phase support)

Examples of the material of the solid-phase support to be used in the present invention include, but are not limited to, a synthetic polymer, such as glass, synthetic resin and synthetic fiber; a metal; a natural polymer, such as natural resin and natural fiber. Examples of a shape of the solid-phase support applicable to the present invention include, but are not limited to, plate-like body, multiwell plate, woven fabric, needle, porous fiber, and beads.

### (Solvent)

Examples of a solvent to be used in the present invention include: an aqueous solution containing at least one compound selected from the group consisting of glucose, sucrose, glycogen, glutamine, arginine, histidine, lysine, threonine, tryptophan, valine, alanine, glycine, proline, and serine; an aqueous solution containing a medium component for culturing cell; and pure water.

### (Printing device)

Examples of a printing device to be used in the present invention include, but are not limited to, ink-jet printer and liquid-handling device.

### (Cell)

For the cell into which the gene sample can be transferred using the transfection device of the present invention, any cell can be used as long as it is a culturable eukaryotic cell, and examples include, but are not limited to, HeLa cell (human cervical cancer).

Hererinbelow, embodiments will be described with reference to the drawings.

### <First embodiment>

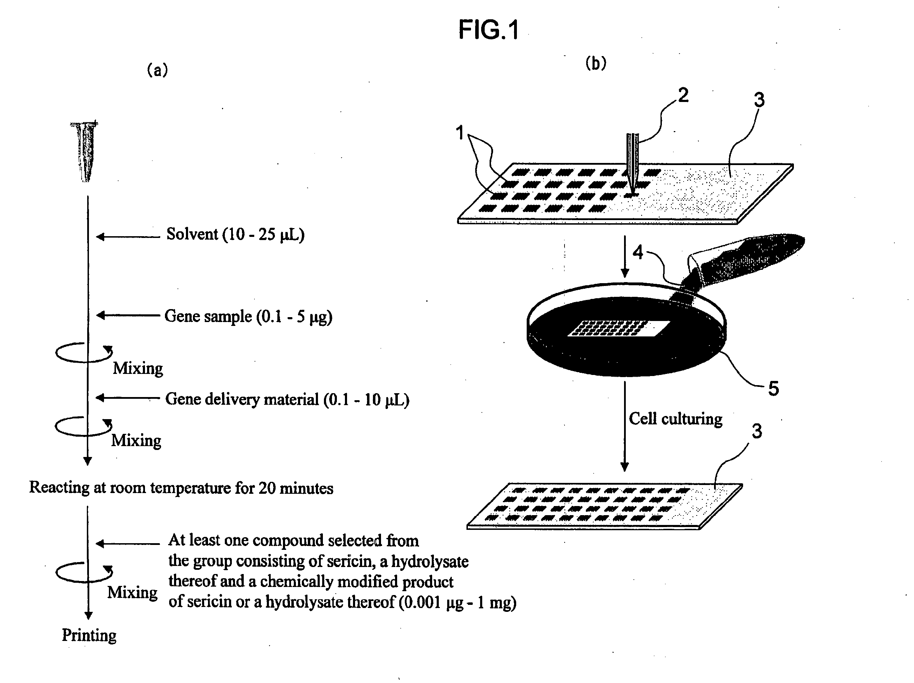

Fig. 1 is a diagram illustrating one example of a method for fabricating a transfection device (glass array) using a glass slide as solid-phase support.

As shown in Fig. 1(a), in an appropriate container, such as Eppendorf tube, the gene sample (e.g. 0.1 µg to 5 µg) is dissolved in the solvent of (e.g. 10 µL to 25 µL), to which the gene delivery material of (e.g. 0.1 µL to 10 µL) is further added, and the mixture is stirred to allow the reaction to proceed under predetermined conditions (e.g. at room temperature for 20 minutes).

After a predetermined reaction time, to the reaction liquid is added at least one compound selected from the group consisting of sericin, a hydrolysate thereof and a chemically modified product of sericin or a hydrolysate thereof in an amount of, for example, 0.001 µg to 1 mg, and the mixture is stirred, to thereby prepare a mixed solution 1.

Next, as shown in Fig. 1(b), using an appropriate printing device 2, the mixed solution 1 is printed on a glass slide 3 as solid-phase support to thereby form spots thereon, which are then allowed to dry.

For performing transfection (gene transfer), as shown in Fig. 1(b), the glass slide 3 having the spots formed thereon is placed in a dish 5, and a cell suspension 4 containing an appropriate type of cell is poured and seeded on the glass slide 3. By incubating the cells, the gene transfer is completed.

It should be noted that cells with gene transfer are observed only in a region where the mixed solution 1 was printed (i.e., spot region).

### <Second embodiment>

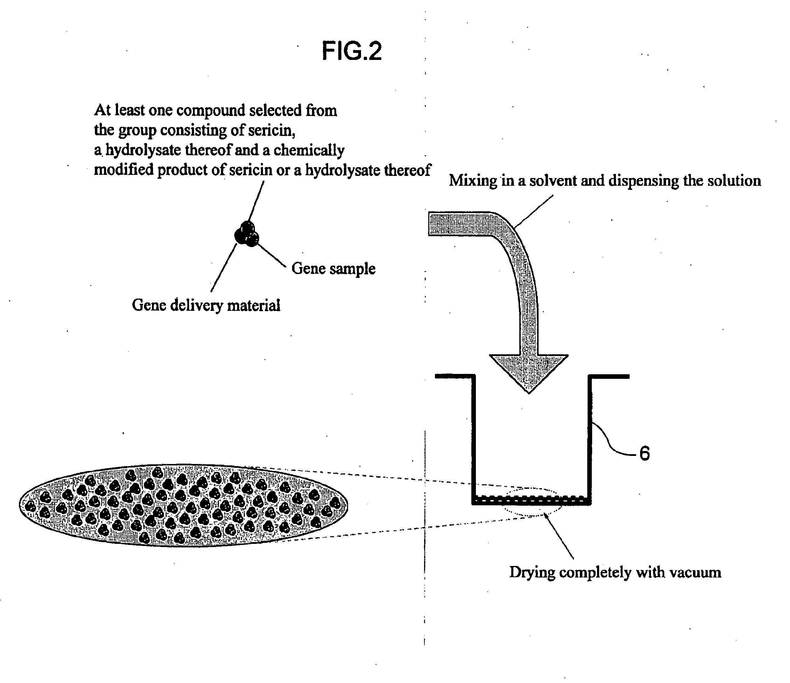

Fig. 2 is a diagram schematically illustrating one example of a method for fabricating the transfection device using a multiwell plate as solid-phase support.

The desired gene sample and an appropriate gene delivery material is dissolved in an appropriate solvent to thereby obtain a solution, and to the solution is further added at least one compound selected from the group consisting of sericin, a hydrolysate thereof and a chemically modified product of sericin or a hydrolysate thereof to thereby prepare a mixed solution. The mixed solution is dispensed into wells 6 of the multiwell plate using a liquid handling device or the like, and then dried with vacuum, to thereby obtain a transfection device.

For performing transfection (gene transfer), a predetermined amount of a cell suspension containing an appropriate type of cell is poured and seeded in each well to which the gene sample and the like has been immobilized. By incubating the cells, the gene transfer is completed.

### (Example 1)

Conventionally, transfection devices have been fabricated using ECM molecule, such as fibronectin. On the other hand, in the present Example, a transfection device was fabricated using the sericin hydrolysate (molecular weight: approximately 5,000 to 40,000, manufactured by Seiren Co. Ltd.), instead of using adhesion molecules or living organism-derived components other than ECM molecule (e.g., albumin complex and albumin).

In the present Example, pEGFP-N1 (plasmid DNA which expresses a green fluorescence protein EGFP) was used as gene sample (DNA). The plasmid DNA (pEGFP-N1), which had been undergone an endotoxin removal process, was dissolved in sterile distilled water containing neither DNase nor RNase, to thereby prepare a 1 µg/µL solution.

In addition, a DMEM (aqueous solution containing components of Dulbecco's modified Eagle medium (medium components for culturing cell)) was used as solvent, and LF2000 (commercially available LipofectAMINE2000 (manufactured by Invitrogen Corporation)) was used as gene delivery material.

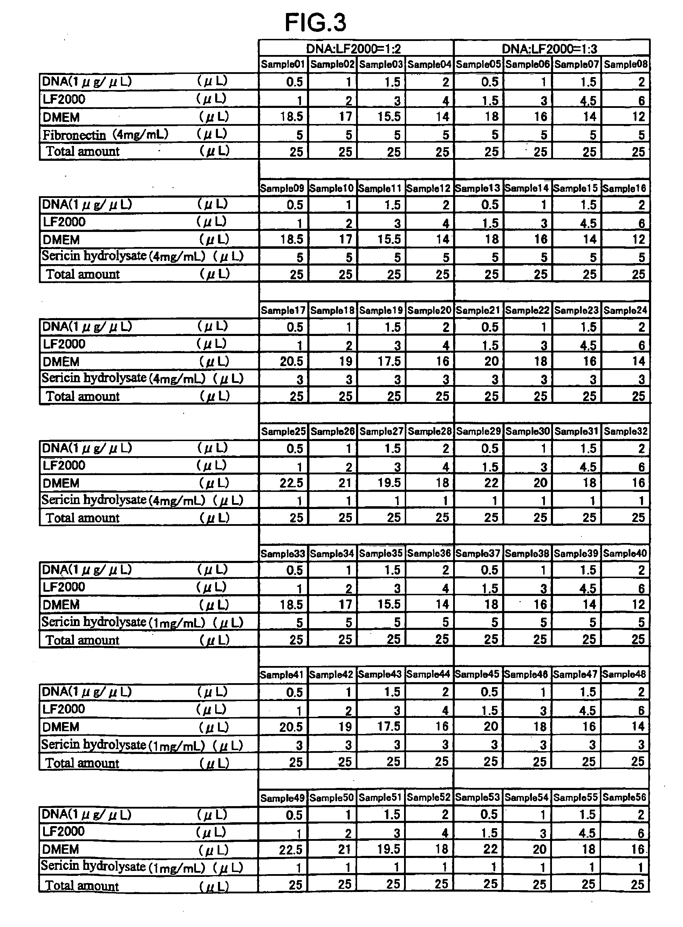

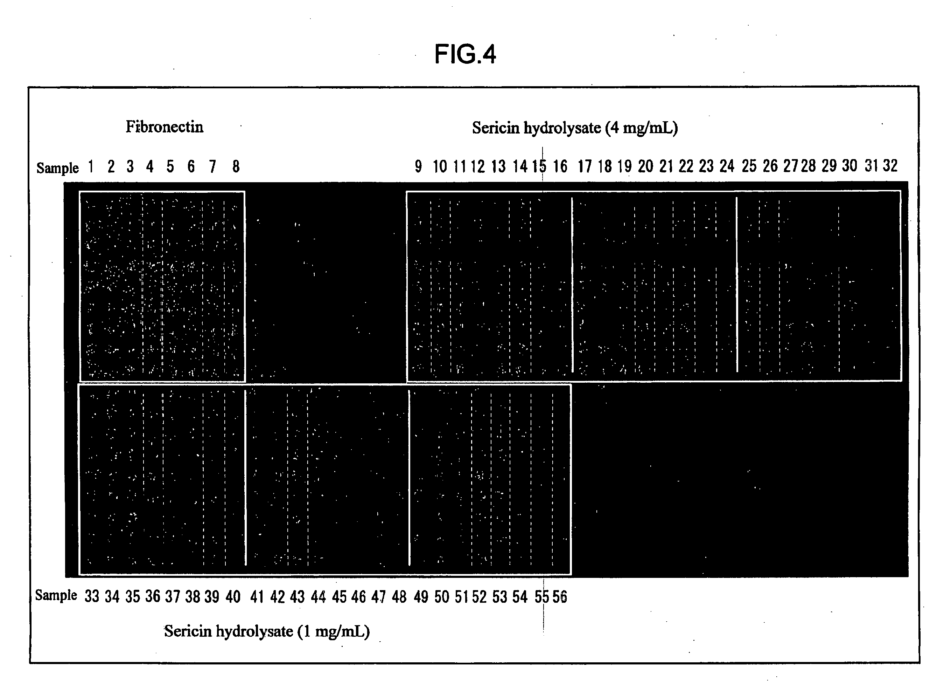

Using the solution compositions shown in Fig. 3, fifty-six types of the mixed solutions (Samples 1 to 56) were prepared. With respect to each of Samples 1 to 8, fibronectin was used instead of the sericin hydrolysate. On a glass slide of the device, nine spots were printed for each sample. Next, HeLa cells (5x10⁴ cell/mL) were seeded, and after 48-hour incubating, a fluorescence image of the green fluorescence protein (EGFP) generated by expression of the transferred gene sample DNA (pEGFP-N1) was obtained, for determining whether or not the transfection took place. The fluorescence image is shown in Fig. 4.

As a result, it was found that the samples using the sericin hydrolysate exhibited equivalent or higher transfection efficiency and localization as compared with the samples using fibronectin.

### (Example 2)

Using a multiwell plate having 1536 wells (NUNC 1536 WELL Assay Plates, No.153613 Greiner 1536 Black flat bottom 782092 (with clear bottom, HI-Base)) as a solid-phase support, a plasmid DNA transfection device (one embodiment of the transfection device of the present invention) was fabricated.

Using the fabricated plasmid DNA transfection device, transfection was performed under conditions described below, to thereby determine whether or not the sericin hydrolysate can effectively function in the plasmid DNA transfection device.

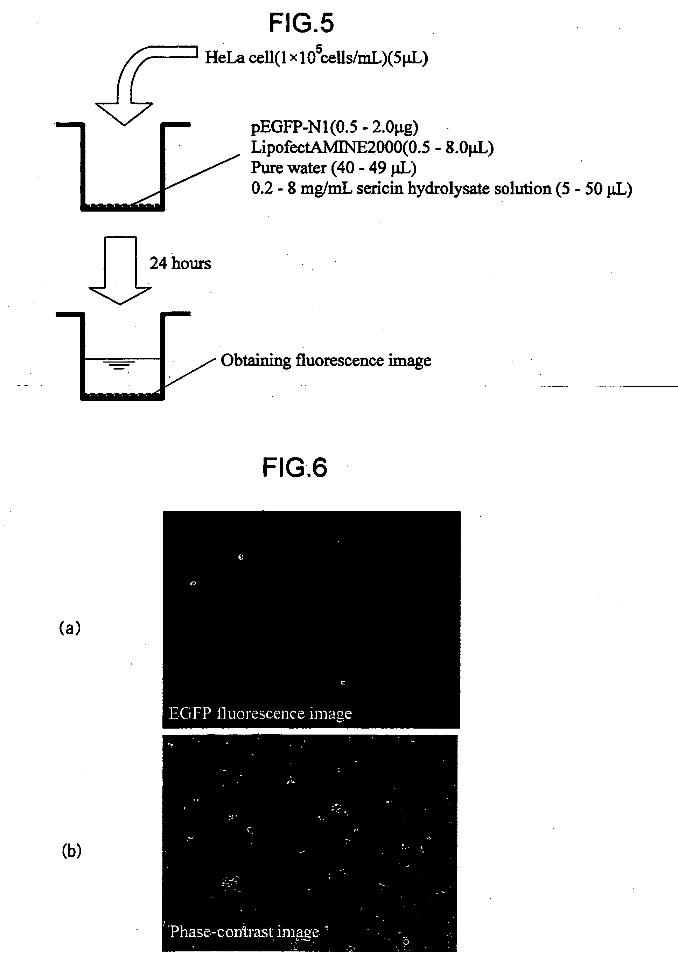

As shown in Fig. 5, the gene sample pEGFP-N1 (0.5 µg - 2.0 µg), the gene delivery material LF2000 (0.5 µL - 8.0 µL), sterilized pure water (40 µL - 49 µL), and a 0.2 mg/mL - 8 mg/mL sericin hydrolysate solution (manufactured by Seiren Co. Ltd.) (5 µL - 50 µL) were mixed, to thereby prepare a mixed solution. The mixed solution was dispensed into wells of the multiwell plate at a volume of 0.5 µL - 4 µL per well using a liquid handling device or the like, and dried completely with vacuum over 2 hours or more.

Then, approximately 500 cells per well of HeLa cells were seeded, and after 24-hours incubating, a fluorescence image of the green fluorescence protein (EGFP) generated by expression of the transferred gene sample DNA (pEGFP-N1) was obtained using an inverted microscope IX-71 (manufactured by Olympus Corporation), for determining whether or not the transfection took place. The fluorescence image is shown in Fig. 6(a).

In addition, with respect to cells incubated for 24 hours, a phase-contrast image was obtained using a phase-contrast microscope, for determining whether or not there is any change in cell morphology caused by cytotoxicity. The phase-contrast image is shown in Fig. 6(b).

As shown in Fig. 6(a), in the plasmid DNA transfection device of the present Example, high transfection efficiency was confirmed, and at the same time, as is apparent from the result shown in Fig. 6(b), in the present Example, especially no change in cell morphology was observed, and no cytotoxicity was recognized.

### (Example 3)

Using a multiwell plate having 1536 wells (NUNC 1536 Well High Base Plates, No.164708) as solid-phase support, an siRNA transfection device (one embodiment of the transfection device of the present invention) was fabricated.

Using the fabricated siRNA transfection device, transfection was performed under conditions described below, to thereby determine whether or not the sericin hydrolysate can effectively function in the siRNA transfection device.

The gene sample pDeRed2-1 used in the present Example is a plasmid DNA having no promoter which thus cannot perform expression, and in the present Example, it was used simply as a carrier for nucleic acids. In addition, this plasmid DNA (pDeRed2-1), which had been undergone an endotoxin removal process, was dissolved in sterile distilled water containing neither DNase nor RNase, to thereby prepare a 1 µg/µL solution.

The gene sample siRNAs used in the present Example are: a negative control siRNA (manufactured by Qiagen: catalog number 1022076) dissolved in a solvent for dissolving siRNA (manufactured by Qiagen) to a concentration of 20 µM; and siCONTROL TOX (manufactured by Dharmacon, Inc.: catalog number D-001500-01-05).

Sterilized pure water was used as solvent, and LF2000 was used as gene delivery material. For cytometry, CellTiter-Blue reagent (diluted 4-fold with D-PBS: manufactured by Promega Corporation) was used.

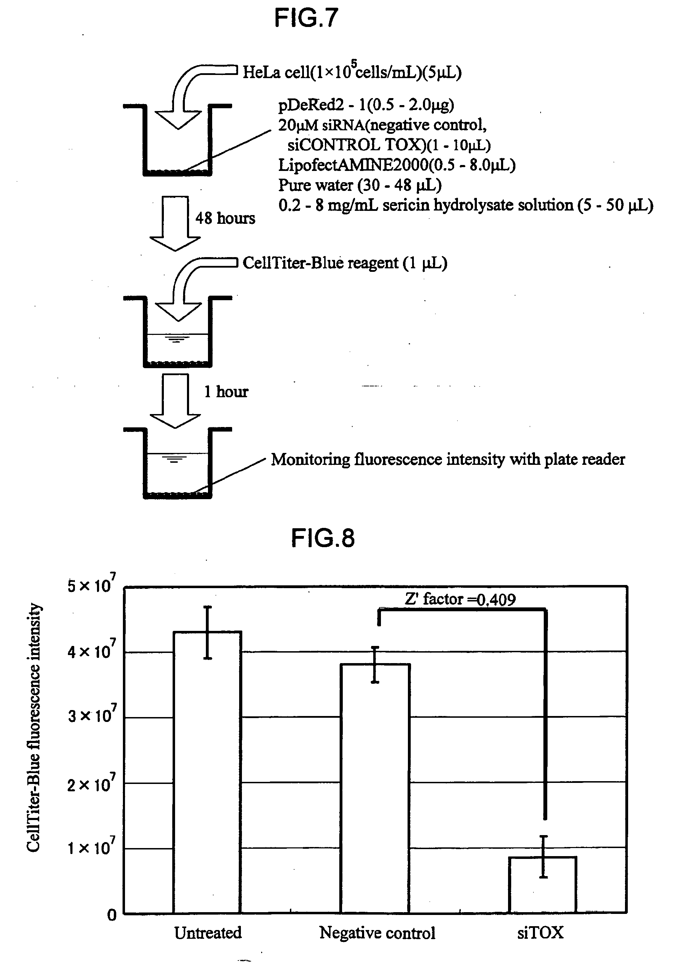

As shown in Fig. 7, there were mixed pDeRed2-1 (0.5 µg - 2.0 µg) and 20 µM siRNA (negative control siRNA or siCONTROL TOX (hereinafter, simply referred to as "siTOX")) (1 µL 10 µL) as gene sample, the gene delivery material LF2000 (0.5 µL - 8.0 µL), pure water (30 µL - 48 µL), and a 0.2 mg/mL - 8 mg/mL sericin hydrolysate solution (manufactured by Seiren Co. Ltd.) (5 µL - 50 µL), to thereby prepare a mixed solution.

The mixed solution was dispensed into wells of the multiwell plate at a predetermined volume (0.5 µL - 4 µL per well) using a liquid handling device or the like, and dried completely with vacuum over 2 hours or more. Then, approximately 500 cells per well of HeLa cells were seeded, and after 48-hour incubating, CellTiter-Blue reagent was added, the plate was left for 1 hour, and then the intensity of fluorescence was monitored with a plate reader.

It should be noted that, for confirming the transfection, the intensity of fluorescence was compared between the well containing the negative control siRNA (well in which transfection is performed using the negative control siRNA) and the well containing siTOX (well in which transfection is performed using siTOX).

In addition, in order to evaluate cytotoxicity in the siRNA transfection device of the present Example, wells containing untreated HeLa cells (approximately 500 cells) with which no transfection was performed were also prepared, and the intensity of fluorescence thereof was also measured together.

The negative control siRNA is siRNA which cannot cause cell death of HeLa cells even when transferred into the cell, while siTOX is siRNA which can cause cell death of HeLa cells when transferred into the cell.

In other words, the cells into which the negative control siRNA is transferred are viable, while the cells into which siTOX is transferred may be killed by induced cell death.

CellTiter-Blue reagent is for monitoring cell viability with fluorescence, based on an ability of living cells to convert a redox dye (resazurin) into a fluorescence product (resorufin), intensity of which is proportional to the viable cell count.

In other words, for confirming the transfection, an inhibition effect of the transferred siRNA on cell proliferation is used as a criterion, and thus the transfection efficiency can be measured by transfecting the cells with the negative control siRNA or siTOX, checking viability of these cells with CellTiter-Blue reagent, and then comparing viable cell counts (intensity of fluorescence) therebetween.

As shown in Fig. 8, in the siRNA transfection device of the present Example, there was little difference in the intensity of fluorescence between a case where transfection was not performed (i.e., cells were seeded into a well containing nothing; in Fig. 8, simply referred to as "untreated") and a case where the negative control siRNA was transfected (in Fig. 8, simply referred to as "negative control"), and almost no cytotoxicity was observed in both cases.

In a case where siTOX was transfected (in Fig. 8, simply referred to as "siTOX"), as compared with the untreated one and the negative control, the intensity of fluorescence (viable cell count) was remarkably small, and thus it is considered that siTOX was surely transferred into the cells, confirming high transfection efficiency.

It should be noted that, in the present Example, the value of Z' factor between the negative control and siTOX was 0.409. This suggests that there was an apparent difference in the intensity of fluorescence between the negative control and siTOX, suggesting that the siRNA transfection device of the present Example exhibits high siRNA transfer efficiency.

### (Example 4)

TRAIL (tumor necrosis factor-related apoptosis inducing ligand) is one type of cytokines identified to have an apoptosis-inducing activity, and it is known that, when cells are incubated in the presence of TRAIL, nearly all of the cells may be killed by apoptosis.

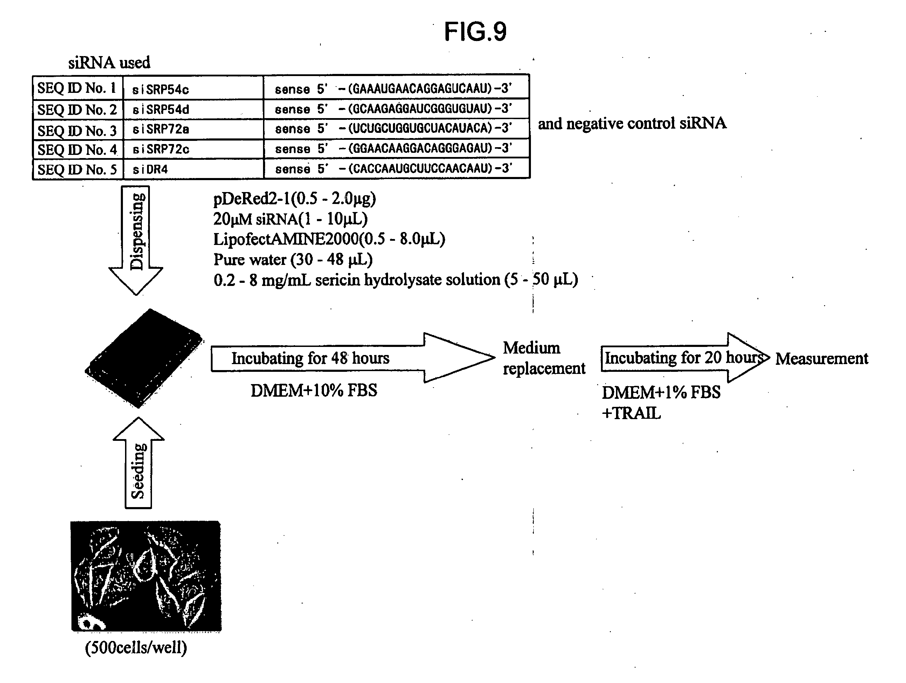

On the other hand, it has been reported by Aza-Blanc et al. that siRNAs shown in SEQ ID No. 1 to 5 in Fig. 9 (siSRP54c, siSRP54d, siSRP72a, siSRP72c, and siDR4) can inhibit apoptosis induced by TRAIL in a liquid-phase transfection screening using 384 wells (see prior art document: Molecular Cell, Vol.12, 627-637, September, 2003).

In the present Example, using the above-described siRNAs shown in SEQ ID No. 1 to 5, an siRNA transfection device was fabricated, and it was confirmed whether or not inhibition effects of these siRNAs on apoptosis can be reproduced.

Using an experimental system shown in Fig. 9, in a 1536-well solid-phase transfection device, the siRNAs shown in SEQ ID No. 1 to 5 were transferred to HeLa cells, and it was determined whether or not there is an inhibition effect on TRAIL-induced apoptosis.

Using a multiwell plate having 1536 wells (NUNC 1536 Well High Base Plates, No.164708) as solid-phase support, an siRNA transfection device (one example of the transfection device of the present invention) was fabricated.

The gene sample pDeRed2-1 used in the present Example is the same as the gene sample in Example 3.

The negative control siRNA as gene sample used in the present Example was prepared by dissolving a negative control siRNA (manufactured by Qiagen: Allstars Negative Control siRNA: catalog number 1027280) in a solvent for dissolving siRNA (manufactured by Qiagen) to a concentration of 20 µM.

Two siRNAs (gene samples) shown in SEQ ID Nos. 1 and 2 are targeting different regions of the base sequence of SRP54 gene which can be expressed during apoptosis, and dissolved in respective solvents for dissolving siRNA (manufactured by Qiagen) each to a concentration of 20 µM.

Two siRNAs (gene sample) shown in SEQ ID Nos. 3 and 4 are targeting different regions of the base sequence of SRP72 gene which can be expressed during apoptosis, and dissolved in respective solvents for dissolving siRNA (manufactured by Qiagen) each to a concentration of 20 µM.

siRNA (gene sample) shown in SEQ ID No. 5 is targeting a portion of the base sequence of DR4 gene which can be expressed during apoptosis, and dissolved in a solvent for dissolving siRNA (manufactured by Qiagen) to a concentration of 20 µM.

Sterilized pure water was used as solvent, and LF2000 was used as gene delivery material.

There were mixed pDeRed2-1 (0.5 µg - 2.0 µg) and 20 µM siRNA (siRNAs shown in SEQ ID No. 1 to 5, or negative control siRNA) (1 µL - 10 µL) as gene sample, the gene delivery material LF2000 (0.5 µL - 8.0 µL), pure water (30 µL - 48 µL) and a 0.2 mg/mL - 8 mg/mL sericin hydrolysate solution (manufactured by Seiren Co. Ltd.) (5 µL - 50 µL), to thereby prepare six types of mixed solutions (6 samples) corresponding to the above-listed different types of siRNA. Each sample was dispensed into wells (8 wells per sample) of the multiwell plate (1536 wells) at a predetermined volume (0.5 µL - 4 µL per well) using a liquid handling device or the like, and dried completely with vacuum over 2 hours or more, to thereby obtain a transfection device.

Then, approximately 500 cells per well of HeLa cells were seeded, and incubated for 48 hours in the presence of DEME and 10% FBS (fetal bovine serum). Subsequently, the medium was replaced with a medium (DMEM and 1% FBS) containing TRAIL (0.5 µg/mL), and after incubating for another 20 hours, CellTiter-Blue reagent (manufactured by Promega Corporation) was added, the plate was left for 1 hour, and then the intensity of fluorescence was monitored with a plate reader.

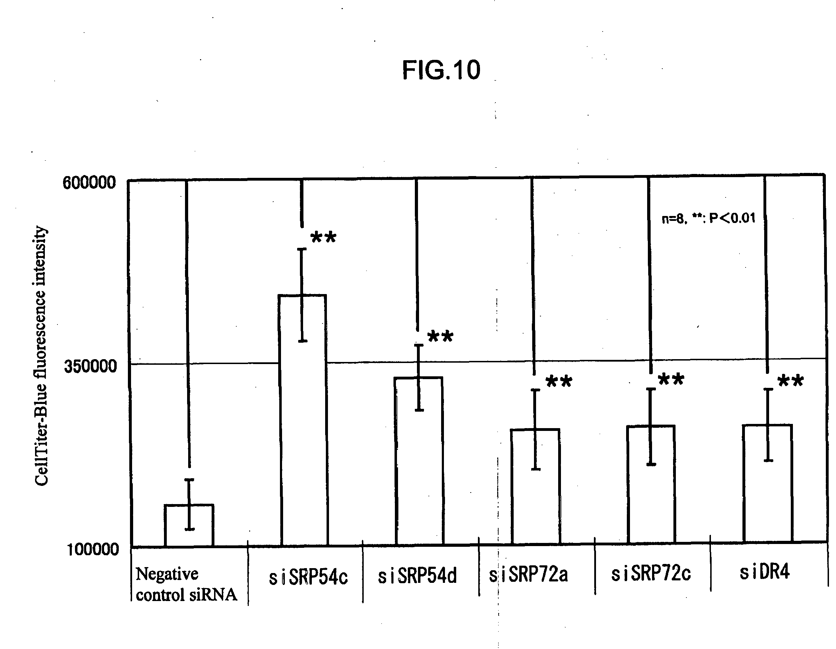

The results are shown in Fig. 10. Since eight specimens were included in one type of sample (n = 8), an average value was calculated for each sample, and the values were plotted into a graph (P < 0.01).

As a result, it was found that with respect to the HeLa cells into which any of the siRNA sequences shown in SEQ ID No. 1 to 5 were transferred (in Fig. 10, simply referred to as "siSRP54c", "siSRP54d", "siSRP72a", "siSRP72c" and "siDR4", respectively), a rate of living cells was significantly increased, while nearly all HeLa cells into which the negative control siRNA was transferred (in Fig. 10, simply referred to as "negative control siRNA") were killed in the presence of TRAIL. From the above, it was confirmed that the siRNA transfer was efficiently preformed in the 1536-well solid phase transfection device.

### (Example 5)

Since living organism-derived molecules, such as fibronectin, will lose their activities after heat sterilization, for sterilization of such molecules, filter sterilization has been used. On the other hand, sericin is **characterized in that** no change occurs in bioactivity after heat sterilization, since it is an extracted component during a refinement process.

Using a sericin hydrolysate solution (1mg/mL: manufactured by Seiren Co. Ltd.) which had been heat-sterilized in an autoclave at 121°C for 20 minutes, siRNA was transferred into HeLa cells on a multiwell plate (1536 wells), and a comparison in activity was made with a case of a sericin hydrolysate solution (1mg/mL: manufactured by Seiren Co. Ltd.) which had been filter-sterilized using a PVDF film (manufactured by Millipore Corporate) having a filter pore diameter of 0.22 µm.

The negative control siRNA as gene sample used in the present Example was prepared by dissolving a negative control siRNA (manufactured by Qiagen: catalog number 1022076) in a solvent for dissolving siRNA (manufactured by Qiagen) to a concentration of 20 µM.

In addition, siRNAs as gene sample were prepared by dissolving siTOX (manufactured by Dharmacon, Inc.: catalog number D-001500-01-05) and KIF11-7 (manufactured by Qiagen: catalog number SI02653770), in respective solvents for siRNA (manufactured by Qiagen), each to a concentration of 20 µM.

Sterilized pure water was used as solvent, and HiPerFect Transfection Reagent (manufactured by Qiagen: catalog number 301702) was used as gene delivery material.

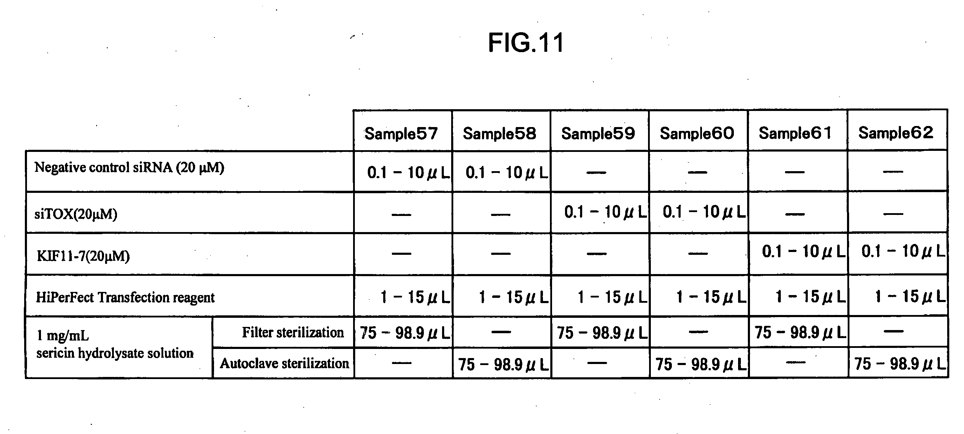

Six different mixed solutions (Samples 57 to 62) having compositions shown in Fig. 11 were prepared, dispensed into wells of the multiwell plate at a predetermined volume (1 µL - 6 µL per well), and dried completely with vacuum to thereby obtain a 1536-well transfection device.

After the vacuum drying, onto the 1536-well transfection device, HeLa cells (1.25 x 10⁵ cells/mL) were seeded at an amount of 4-µL per well, and incubated in a carbon dioxide incubator for 48 hours. For cytometry, CellTiter-Blue reagent (manufactured by Promega Corporation) was used, and effects of the siRNA-transferred cells on cell proliferation were evaluated.

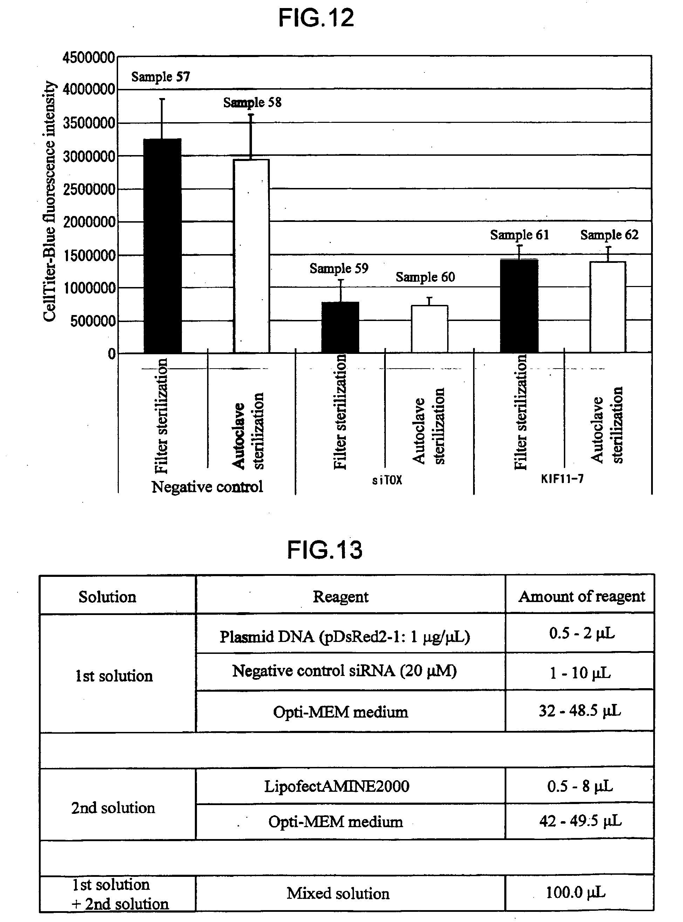

As a result, as shown in Fig. 12, in a case where the negative control siRNA was transferred into cells (in Fig. 12, simply referred to as "negative control"), neither the filter-sterilized sericin hydrolysate solution nor the autoclave-sterilized sericin hydrolysate solution showed no notable cytotoxicity. On the other hand, in a case where siTOX and KIF11-7, which had been confirmed to have a suppressive effect on cell proliferation, were transferred into cells (in Fig. 12, simply referred to as "siTOX" and "KIF11-7", respectively), both the filter-sterilized and autoclave-sterilized sericin hydrolysate solutions showed an apparent inhibition effect on cell proliferation. From these results, it was found that there is no change in the activity of sericin (enhancing nucleic acid transfer efficiency) even after the autoclave sterilization.

### (Example 6)

The solid phase gene transfer includes a step of drying the solution, after printing the solution on a support. During the drying step, components dissolved in the solution may be deposited, and in some cases it may suppress cell proliferation, i.e., generate cytotoxicity. In the case of the transfection device containing fibronectin, there have been sometimes observed that the print solution composition generates cytotoxicity.

It has been known that sericin has a cell-protective effect. It was examined whether sericin has an alleviating effect on cytotoxicity in the transfection device, using fibronectin for comparison.

The gene sample pDeRed2-1 used in the present Example is the same as the gene sample in Example 3.

The siRNA as gene sample used in the present Example was a negative control siRNA (manufactured by Qiagen: catalog number 1022076) dissolved in a solvent for dissolving siRNA (manufactured by Qiagen) to a concentration of 20 µM.

Sterilized pure water and an Opti-MEM medium (manufactured by Invitrogen Corporation) were used as solvent, and LF2000 was used as gene delivery material. For cytometry, CellTiter-Blue reagent (manufactured by Promega Corporation) was used.

With respect to the compositions shown in Fig. 13, there were prepared a first solution containing the gene samples (pDeRed2-1 and negative control siRNA) and the Opti-MEM medium and a second solution containing the gene delivery material (LipofectAMINE2000) and the Opti-MEM medium, and then the first solution and the second solution were mixed to thereby prepare a mixed solution.

With a sericin hydrolysate solution (4 mg/mL) which had been dissolved into sterile distilled water, or a fibronectin solution (4 mg/mL) which had been dissolved into sterile distilled water, the mixed solution was diluted at a predetermined rate (1.5 to 20 folds), dispensed into wells of the multiwell plate (1536 wells) at a predetermined volume (1 µL - 6 µL) per well, and dried completely with vacuum to thereby obtain a 1536-well transfection device.

After the vacuum drying, onto the 1536-well transfection device, HeLa cells (1.25 x 10⁵ cells/mL) were seeded at an amount of 4 µL per well, and incubated in a carbon dioxide incubator for 48 hours. For cytometry, CellTiter-Blue reagent (manufactured by Promega Corporation) was used, and effects of the nucleic acid-transferred cells on cell proliferation were evaluated.

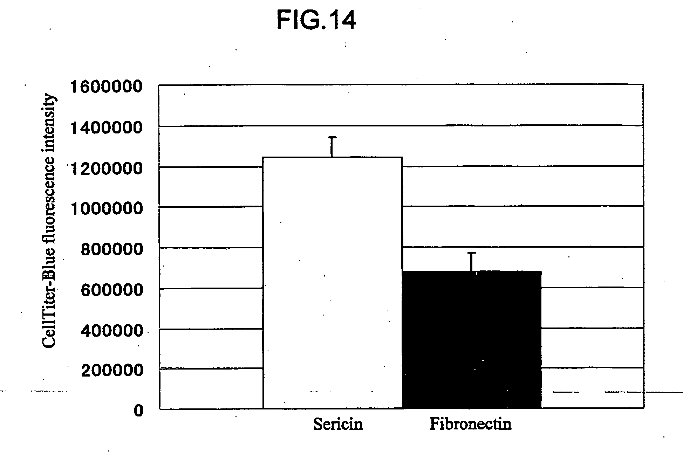

As a result, as shown in Fig. 14, as compared with the transfection device in which dilution was made using a fibronectin solution (in Fig. 14, simply referred to as "fibronectin"), an apparent alleviating effect on cytotoxicity was observed in the transfection device in which dilution was made using the sericin hydrolysate solution (in Fig. 14, simply referred to as "sericin").

### INDUSTRIAL APPLICABILITY

The present invention is applicable to a transfection device for transferring an exogenous gene sample (for example, single-stranded or double-stranded deoxyribonucleic acid (DNA), ribonucleic acid (RNA), aptamer and chemically modified derivatives of thereof) into an animal cell.

## Claims

1. A transfection device having a mixture *(1)* immobilized on a solid-phase support *(3)*, the mixture *(1)* comprising:
- a gene sample;
- a gene delivery material; and
- a mixture containing *a sericin hydrolysate having a molecular weight of approximately 5,000 to 40,000.*

2. The transfection device according to claim 1, wherein the gene sample is at least one member selected from the group consisting of single-stranded or double-stranded deoxyribonucleic acid, ribonucleic acid, aptamer, and a chemically modified derivative thereof.

3. The transfection device according to claim 1 or 2, wherein the gene delivery material comprises at least one member selected from the group consisting of a cationic polymer, a cationic lipid, and a mineral.

4. The transfection device according to any one of claims 1 to 3, wherein the mixture (1) further comprises one of the followings:
- at least one compound selected from the group consisting of glucose, sucrose, glycogen, glutamine, arginine, histidine, lysine, threonine, tryptophan, valine, alanine, glycine, proline, and serine;
- a medium component for culturing cell; and
- pure water.

5. The transfection device according to any one of claims 1 to 4, wherein a material of the solid-phase support (3) is selected from the group consisting of glass, a synthetic polymer, a metal, and a natural polymer.

## Patentansprüche

1. Transfektionsvorrichtung, die ein Gemisch (1) aufweist, das auf einem Festphasenträger (3) immobilisiert ist, wobei das Gemisch (1) Folgendes umfasst:
- eine Genprobe;
- ein Genabgabematerial; und
- ein Gemisch, das ein Sericinhydrolysat mit einem Molekulargewicht von etwa 5.000 bis 40.000 enthält.

2. Transfektionsvorrichtung gemäß Anspruch 1, wobei die Genprobe mindestens ein Teil ist, das aus der Gruppe ausgewählt ist, die aus einzel- oder doppelsträngiger Desoxyribonukleinsäure, Ribonukleinsäure, Aptamer und einem chemisch modifizierten Derivat davon besteht.

3. Transfektionsvorrichtung gemäß Anspruch 1 oder 2, wobei das Genabgabematerial mindestens ein Teil aufweist, das aus der Gruppe ausgewählt ist, die aus einem kationischen Polymer, einem kationischen Lipid und einem Mineral besteht.

4. Transfektionsvorrichtung gemäß einem der Ansprüche 1 bis 3, wobei das Gemisch (1) zusätzlich eines der Folgenden aufweist:
- mindestens eine Verbindung, die aus der Gruppe ausgewählt ist, die aus Glucose, Saccharose, Glycogen, Glutamin, Arginin, Histidin, Lysin, Threonin, Tryptophan, Valin, Alanin, Glycin, Prolin und Serin besteht;
- eine Mediumkomponente zum Kultivieren von Zellen; und
- reines Wasser.

5. Transfektionsvorrichtung gemäß einem der Ansprüche 1 bis 4, wobei ein Material des Festphasenträgers (3) aus der Gruppe ausgewählt ist, die aus Glas, einem synthetischen Polymer, einem Metall und einem natürlichen Polymer besteht.

## Revendications

1. Dispositif de transfection ayant un mélange (1) immobilisé sur un support en phase solide (3), le mélange (1) comprenant:
- un échantillon de gène;
- une matière d'administration de gène; et
- un mélange contenant un hydrolysat de séricine ayant une masse moléculaire d'approximativement 5 000 à 40 000.

2. Dispositif de transfection selon la revendication 1, dans lequel l'échantillon de gène est au moins un élément sélectionné dans le groupe constitué d'un acide désoxyribonucléique simple brin ou double brin, d'un acide ribonucléique, d'un aptamère, et d'un dérivé chimiquement modifié de ceux-ci.

3. Dispositif de transfection selon la revendication 1 ou 2, dans lequel la matière d'administration de gène comprend au moins un élément sélectionné dans le groupe constitué d'un polymère cationique, d'un lipide cationique, et d'un minéral.

4. Dispositif de transfection selon l'une quelconque des revendications 1 à 3, dans lequel le mélange (1) comprend en outre l'un de:
- au moins un composé sélectionné dans le groupe constitué du glucose, du saccharose, du glycogène, de la glutamine, de l'arginine, de l'histidine, de la lysine, de la thréonine, du tryptophane, de la valine, de l'alanine, de la glycine, de la proline, et de la sérine;
- un composant de milieu pour culture cellulaire; et
- de l'eau pure.

5. Dispositif de transfection selon l'une quelconque des revendications 1 à 4, dans lequel une matière du support en phase solide (3) est sélectionnée dans le groupe constitué du verre, d'un polymère synthétique, d'un métal, et d'un polymère naturel.
